# EUROPÄISCHE PATENTSCHRIFT

(11) **EP 1 360 968 B1**
(45) Veröffentlichungstag und Bekanntmachung des Hinweises auf die Patenterteilung: **02.11.2006**
(21) Anmeldenummer: 03010074.7
(22) Anmeldetag: 03.05.2003
(51) Int. Cl.: A61M 5/165, A61M 5/158

(54) **Injektionsanordnung**
Injection system
Système d'injection

(30) Priorität: 06.05.2002 DE 20207083 U; 24.05.2002 DE 20208105 U
(43) Veröffentlichungstag der Anmeldung: 12.11.2003
(73) Patentinhaber: Scheu, Rolf Rainer, 60488 Frankfurt/Main (DE)
(72) Erfinder: Scheu, Rolf Rainer, 60488 Frankfurt/Main (DE)
(74) Vertreter: Müller-Gerbes, Margot

(56) Entgegenhaltungen:
- EP-A- 0 998 978
- US-A- 3 631 654
- US-A- 4 161 504
- US-A- 4 200 096
- US-A- 4 453 927

## Beschreibung

Die Erfindung betrifft eine Injektionsanordnung zum Injizieren von Injektionslösungen, welche aus pulverförmigen Arzneimitteln unmittelbar vor Gebrauch in sterilem Wasser zubereitet und mittels einer Spritze verabreicht werden, umfassend eine Flügelkanüle mit einer Hohlnadel und einem Nadelhalter mit Flügeln sowie einen mit der Flügelkanüle mittels eines flexiblen Verbindungsschlauches verbundenen weiblicher Luer-Lock-Konnektor mit einem Einsteckbereich für den Verbindungsschlauch und einem Konnektionsbereich für das Ansetzen der mit Injektionslösung gefüllten Spritze und einer vom Einsteckbereich bis zum Konnektionsbereich des Luer-Lock-Konnektors durchgehenden Bohrung, in die ein für die Injektionslösung durchlässiges poröses Filterelement eingesetzt ist.

Derartige Injektionsanordnungen werden beispielsweise zur Verabreichung von Gerinnungsmitteln, welche in ein Blutgefäß eines Patienten injiziert werden, benötigt. Gerinnungsmittel liegen in Pulverform vor und werden erst unmittelbar vor Gebrauch in Wasser gelöst, um dann verabreicht zu werden, da diese Arzneimittel in gelöster Form keine lange Haltbarkeitszeit aufweisen.

Für das Injizieren von Injektionslösungen, die erst unmittelbar vor Gebrauch mittels sterilem Wasser in eine Lösung überführt werden, ist ein Schutz gegen Verunreinigungen erforderlich, d.h. die frisch hergestellte Injektionslösung ist vor Verabreichung an den Patienten einem Reinigungsvorgang zu unterziehen, um möglicherweise Fremdkörper, wie Feststoffpartikel aus Verunreinigungen zu entfernen.

Aus der US-A-4 200 096 ist eine Injektionsanordnung bekannt, bei der ein scheibenförmiges Filterelement lose und damit herausnehmbar zwischen dem proximalen Ende einer Injektionsnadel und einem Konnektor eingeklemmt wird, um einerseits bei der Gefäßpunktion ein Ausfließen von Blut aus der Injektionsnadel zu unterbinden und andererseits die zu injizierende Flüssigkeit zu filtrieren.

Der Erfindung liegt die Aufgabe zugrunde, das Verabreichen mittels Injektion von aus pulverförmigen Arzneimitteln unmittelbar vor Gebrauch hergestellten Injektionslösungen mit einer leicht zu handhabenden Injektionsanordnung zu ermöglichen, wobei die Reinigung bzw. Reinhaltung der Injektionslösung gleichzeitig erfolgt. Da derartige Injektionsanordnungen Einmalartikel sind, soll die Injektionsanordnung auch einfach und wirtschaftlich und kostengünstig aufgebaut und herstellbar sein.

Der Einsatz von Filtern für medizinische Anwendungen zwecks Dekontamination ist bekannt. Beispielsweise ist aus der DE 24 01 782 A eine Filtervorrichtung für medizinische Infusions- und Injektionseinrichtungen mit einem Nadelhalter mit Hohlnadel und einer Spritze bekannt, bei welcher ein zylindrischer becherförmiger poröser Filter aus gesintertem Metallpulver vorgesehen ist. Der Filter ist auf einer Seite in den mit einer durchgehenden Bohrung versehenen Nadelhalter eingesetzt und die Hohlnadel auf der anderen Seite in der Bohrung des Nadelhalters befestigt. Die Spritze wird dann in die mit dem Filter ausgestattete Bohrung des Nadelhalters eingeführt, wobei der Filter teilchenförmige Verschmutzungen aus dem dem Patienten zugeführten parenteralen Flüssigkeiten ausfiltern soll. Aus der US 4,435,176 ist ebenfalls eine Injektionseinrichtung umfassend eine Hohlnadel und einen Nadelhalter und eine Spritze analog zur DE 24 01 782 A bekannt, bei welcher jedoch ein plattenförmiger Filter verwendet ist. Diese bekannten Injektionseinrichtungen bilden eine kompakte Einheit, wobei die Spritze unmittelbar mit der Hohlnadel über den Nadelhalter verbunden ist und damit unmittelbar an der Einstichstelle des Patienten benachbart ist und das Handling erschwert.

Aus der DE 297 201 82 U1 ist eine weitere Injektionseinrichtung mit einer Spritze und einem Nadelhalter mit Hohlnadel bekannt, bei welcher zwischen dem Nadelhalter und der Spritze ein als separates Teil ausgebildeter Filter eingesetzt wird. Hier bilden Hohlnadel, Nadelhalter, Filter und Spritze eine kompakte formstabile Einheit, wobei die Spritze ebenfalls patientennah ist und die kompakte formstabile Einheit das Handling für den Patienten selbst erschwert.

Aus den US 5,603,792 und 5,500,003 ist ein Schutz in Form einer Membran für einen Druckaufnehmer zur Messung des Blutdrucks eines Patienten bei Blutdialyseverfahren bekannt. Die Membran übernimmt gleichzeitig die Funktion einer Steril-Barriere, welche sowohl den Patienten als auch das Dialyseequipment vor der Gefahr der Kontamination mit vireninfiziertem Blut schützt. Die Membran ist hierbei zwischen zwei Flanschen aus Polycarbonat eingeschweißt, die an dem patientenseitigen Ausgang eines weiblichen Luer-Lock-Konnektors dauerhaft angebracht sind, an dessen anderem Ausgang das Dialyseequipment zwecks Druckmessung anschließbar ist.

Die der Erfindung zugrundeliegende Aufgabe wird durch Weiterbildung der gattungsgemäßen Injektionsanordnung gemäß den Merkmalen des Patentanspruches 1 gelöst.

Erfindungsgemäß wird zur Reinigung der zu injizierenden Injektionslösung ein für die Injektionslösung durchlässiges poröses Filterelement in der durchgehenden Bohrung des weiblichen Luer-Lock-Konnektors vorgesehen, bei dem der weibliche Luer-Lock-Konnektor integral mit dem Filterelement verbunden ist. Hierzu wird vorgeschlagen, daß ein erstes, den Einsteckbereich bildendes Spritzgußteil aus einem thermoplastischen Kunststoff vorgesehen ist, auf dessen Eingangsseite das Filterelement aufgelegt und durch Aufspritzen eines zweiten, den Konnektionsbereich bildenden Spritzgußteils aus dem gleichen oder einem anderen thermoplastischen Kunststoff wie der Einsteckbereich eingebettet ist und beide Spritzgußteile zu dem Luer-Lock-Konnektor vereinigt sind. Die Spritze kann dann unmittelbar auf den weiblichen Luer-Lock-Konnektor im Konnektionsbereich angesetzt werden und die aus der Spritze ausgedrückte Injektionslösung wird durch das poröse Filterelement hindurchgedrückt und gelangt von dem Luer-Lock-Konnektor über den Verbindungsschlauch in die mit der Flügelkanüle ausgestattete Hohlnadel zum Patienten.

Erfindungswesentlich ist die Ausbildung des Filterelementes mit einer Durchlässigkeit, welche das Strömen der Injektionslösung nicht behindert, gleichzeitig jedoch in der Injektionslösung noch enthaltene Feststoffteilchen zurückhält und verhindert, daß diese das Filterelement durchdringen. Erfindungsgemäß ist das Filterelement mit ausreichender Durchlässigkeit und einer Porengröße, die einerseits nicht unterschritten werden darf, um das Strömen der Injektionslösung nicht zu verhindern und andererseits nicht überschritten werden darf, um die Feststoffteilchen aufzufangen, auszustatten. Bevorzugt weist das Filterelement Poren mit einem durchschnittlichen Durchmesser von 2 - 100 µm auf und bevorzugt Poren mit einem mittleren Durchmesser von 5 - 20 µm.

Vorteilhafte Ausgestaltungen und Weiterbildungen der Erfindung sind den Unteransprüchen entnehmbar.

Die erfindungsgemäß ausgestattete Injektionsanordnung gestattet eine einfache Handhabung für das Injizieren von Injektionslösungen durch den Patienten selbst, da mittels der Flügel der Flügelkanüle eine Führung der Nadel und Punktion des vorgesehenen Blutgefäßes einfach erfolgen kann. Der mittels des Verbindungsschlauches flexibel mit der Flügelkanüle verbundene weibliche Luer-Lock-Konnektor gestattet die Verbindung zu einer Spritze mit Luer-Lock-Anschluß oder Luer-Anschluß, die das zu injizierende Medikament als Injektionslösung enthält, wobei das im Luer-Lock-Konnektor angeordnete Filterelement zuverlässig Feststoffe zurückhält, die beispielsweise während der Bereitung der Injektionslösung in der anzuschließenden Spritze zurückbleiben. Dies ist beispielsweise bei der Bereitung von Gerinnungsmitteln für Bluter-Patienten häufig der Fall, da das Medikament zunächst in Pulverform vorliegt und mittels Wasser zu einer injizierbaren Lösung aufbereitet wird, bei der jedoch Feststoffe zurückbleiben können, die zuverlässig vom Filterelement zurückgehalten werden.

Die erfindungsgemäße Injektionsanordnung ermöglicht eine besonders leichte Handhabung und Verabreichung eines Medikamentes, welche ggf. vom Patienten selbst vorgenommen werden kann.

Insbesondere gestattet die erfindungsgemäße Injektionsanordnung die Anwendung von Medikamenten, welche vor ihrer Verabreichung in Pulverform vorliegen und erst unmittelbar vor Gebrauch zu einer Injektionslösung durch Zusatz von Wasser zubereitet werden.

Hierzu kann beispielsweise eine Einweg-Glas-Spritze verwendet werden, die mit dem pulverförmigen Medikament vorbefüllt ist. Zur Verabreichung des Medikamentes wird diese Spritze zunächst mit sterilem Wasser aufgezogen und dann der Inhalt gut durchgeschüttelt, um so eine Injektionslösung aus Medikament und Wasser zu erhalten. Dann wird die Spritze mit dem Konnektionsbereich des Luer-Lock-Konnektors der erfindungsgemäßen Injektionsanordnung verbunden und die Injektionslösung kann über die in ein Blutgefäß des Patienten eingesteckte Hohlnadel der Flügelkanüle verabreicht werden. Eventuell in der Injektionslösung noch enthaltene Feststoffteilchen werden hierbei vom Filterelement, das in dem Luer-Lock-Konnektor eingebaut ist, zuverlässig zurückgehalten.

Die erfindungsgemäße Injektionsanordnung ist durch die Flügelkanüle leicht handhabbar und führbar und es ist auch eine leichte Entkoppelung der Flügelkanüle von der am Luer-Lock-Konnektor konnektierten Spritze mittels des flexiblen Verbindungsschlauches ermöglicht.

Ein wesentliches Merkmal der Erfindung ist das Anordnen des Filterelementes innerhalb des Luer-Lock-Konnektors. Bevorzugt wird das Filterelement im Übergang von dem Einsteckbereich des Luer-Lock-Konnektors zu dem Konnektionsbereich des Luer-Lock-Konnektors für die Spritze angeordnet, wo die durchgehende Bohrung des Luer-Lock-Konnektors sich in Richtung auf den Konnektionsbereich hin erweitert und einen stufenförmigen Absatz bildet. An diesem Absatz kann das Filterelement anliegen. Das Filterelement ist vorzugsweise plattenförmig ausgebildet.

Das Filterelement wird innerhalb des Luer-Lock-Konnektors in seiner Position dauerhaft fixiert, beispielsweise ist das Filterelement entlang seines Außenumfanges in den Luer-Lock-Konnektor eingelagert. Dies kann beispielsweise in der Weise erfolgen, daß der Luer-Lock-Konnektor als Spritzgußteil aus einem thermoplastischen Kunststoff hergestellt wird und das Filterelement beim Herstellen des Spritzgußteiles in die Form eingelegt und partiell umspritzt ist, d.h. in den thermoplastischen Kunststoff des Luer-Lock-Konnektors partiell eingebettet ist, insbesondere entlang seines Außenumfanges. Der die durchgehende Bohrung des Luer-Lock-Konnektors abdeckende Bereich des Filterelementes bleibt frei und damit durchlässig für die zu injizierende Injektionslösung.

Das Filterelement, welches innerhalb des Luer-Lock-Konnektors angeordnet ist, ist bevorzugt aus einem Gewebe auf Basis von Polyamid, wie Nylon, ausgebildet, wobei die Porengröße/offene Maschenfläche des Filterelementes im Bereich von 2 - 100 µm gewählt ist. Partikel mit größerer Fläche als der offenen Maschenfläche des Gewebes des Filterelementes werden von diesem zurückgehalten.

Es ist auch möglich, ein als Scheibe ausgebildetes Filterelement aus gesintertem Kunststoffgranulat oder Metallpulver innerhalb des Konnektors anzuordnen. Die ausgewählte Porengröße des Filterelementes gewährleistet, daß die Injektionslösung aus der am Luer-Lock-Konnektor angeschlossenen Spritze mit einem gewöhnlichen Daumendruck eines Patienten noch problemlos appliziert werden kann und gleichzeitig die kritischen zu erwartenden Feststoffpartikel zuverlässig von dem Filterelement zurückgehalten werden.

Darüber hinaus können als Filterelement auch Membranen aus Kunststoffolie eingesetzt werden, sofern sie mit Poren in ausreichender Zahl und Größe von 2 -100 µm ausgestattet sind, um die Injektionslösung hindurch zu lassen.

Der für die erfindungsgemäße Injektionsanordnung vorgesehene Verbindungsschlauch ist aus einem flexiblen Kunststoff, wie beispielsweise Weich-PVC oder einem thermoplastischen/elastischen Polyurethan hergestellt und weist eine Länge von 30 - 400 mm, bevorzugt 300 - 400 mm auf. Der Außendurchmesser des Verbindungsschlauches liegt bei 1,0 - 2,5 mm bei einem Innendurchmesser von 0,2 - 1,5 mm. Die Abmessungen des Verbindungsschlauches sind so gewählt, daß ein möglichst kleines Innenvolumen, welches einen Totraum für die zu injizierende Injektionslösung bildet, erhalten wird, wobei gleichzeitig die Injektion durch den Verbindungsschlauch mit noch vertretbarem Kraftaufwand durchführbar sein soll. Der Verbindungsschlauch wird in die Bohrung des Einsteckbereiches des Luer-Lock-Konnektors eingeführt und dort dauerhaft befestigt, beispielsweise mittels HF- oder Ultraschall-Verschweißung, je nach eingesetztem Material für den Verbindungsschlauch und den Luer-Lock-Konnektor. Darüber hinaus ist es möglich, den Luer-Lock-Konnektor an seinem Einsteckbereich außenseitig mit zwei in einer Ebene liegenden angeformten Flügeln auszustatten.

Der Luer-Lock-Konnektor für die erfindungsgemäße Injektionsanordnung ist bevorzugt aus einem thermoplastischen Kunststoff, wie Acrylbutadienstyrol, Polycarbonat oder Polymethacrylat im Spritzgußverfahren hergestellt. Hierbei wird das im Inneren des Luer-Lock-Konnektors angeordnete Filterelement integral bei der Herstellung des Konnektors mit eingelegt und teilweise umspritzt. Erfindungsgemäß ist der weibliche Luer-Lock-Konnektor mit Filterelement aus einem ersten den Einsteckbereich bildenden Spritzgußteil aus einem thermoplastischen Kunststoff gebildet, bei dem das auf dessen Eingangsseite aufgelegte Filterelement durch Aufspritzen eines zweiten, den Konnektionsbereich bildenden Spritzgußteils aus dem gleichen oder einem anderen thermoplastischen Kunststoff wie der Einsteckbereich eingebettet ist und beide Spritzgußteile zu dem Konnektor vereinigt sind.

Es sind auch andere Arten der Einbringung des Filterelementes in die Bohrung des Luer-Lock-Konnektors möglich, die nicht zum Gegenstand der Erfindung gehören. Beispielweise kann der Luer-Lock-Konnektor als Spritzgußteil aus einem thermoplastischen Kunststoff hergestellt sein und das Filterelement wird nachträglich in die durchgehende Bohrung des Luer-Lock-Konnektors eingesetzt und hierin dauerhaft befestigt, beispielsweise durch HF-Verschweißung.

Die an der Flügelkanüle der erfindungsgemäßen Injektionsanordnung vorgesehene Hohlnadel kann eine aktive Länge, mit welcher sie über den Nadelhalter vorsteht, von 15 - 20 mm bei einem Außendurchmesser von 0,4 - 1,2 mm, vorzugsweise 0,45 - 0,6 mm aufweisen, wobei der Innendurchmesser 0,25 - 0,38 mm betragen kann.

Die Hohlnadel der Injektionsanordnung wird in ihrem über den Nadelhalter vorstehenden aktiven Bereich mittels einer abziehbaren Schutzhülle vor dem Gebrauch geschützt. Darüber hinaus ist es möglich, die Hohlnadel, insbesondere die Spitze derselben nach Gebrauch der Injektionsanordnung ebenfalls wieder mit einem Abdeckschutz zu versehen. Dieser Abdeckschutz kann beispielsweise aus einem an dem Nadelhalter angeordneten Abdeckelement oder Klappschutz bestehen. Es ist auch möglich, die Flügelkanüle so auszubilden, daß die Hohlnadel nach Gebrauch in den

Nadelhalter zurückziehbar ist oder der Nadelhalter mit einem auf die Hohlnadel vorschiebbaren Abdeckteil ausgestattet ist.

Weitere Einzelheiten der erfindungsgemäßen Injektionsanordnung werden nachfolgend anhand von Ausführungsbeispielen in der Zeichnung erläutert. Es zeigen:
- Figur 1: die Injektionsanordnung gemäß der Erfindung
- Figur 2: in vergrößerter Darstellung den Nadelhalter gemäß Figur 1
- Figur 3: in vergrößerter Darstellung die Flügelkanüle gemäß Figur 1
- Figur 4: den weiblichen Luer-Lock-Konnektor mit Filterelement gem. Figur 1 in vergrößerter Darstellung
- Figur 5: eine Variante des Luer-Lock-Konnektors gemäß Figur 4
- Figur 6: einen weiblichen Luer-Lock-Konnektor mit Flügeln für die Injektionsanordnung nach Figur 1
- Figur 7: die Injektionsanordnung gemäß der Erfindung einschließlich Spritze in der Anwendung in schematischer Darstellung

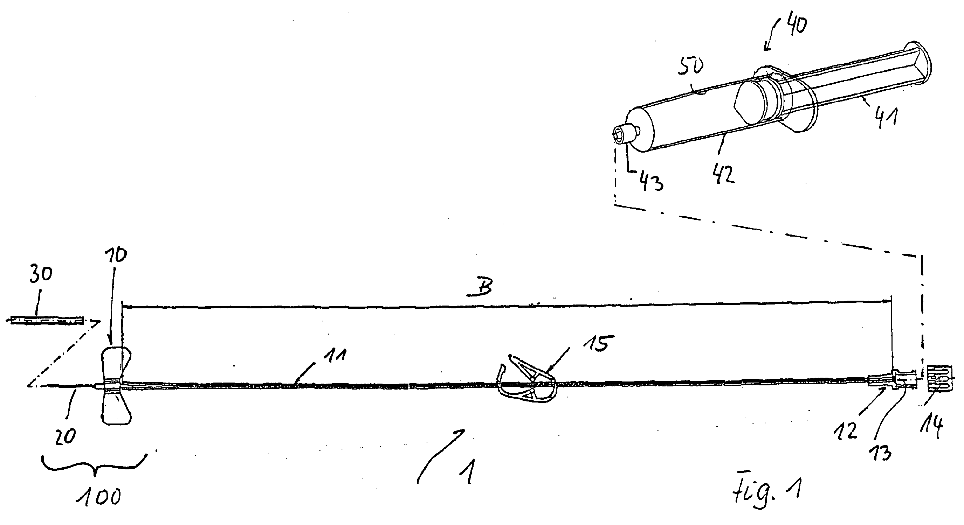

Die in der Figur 1 dargestellte Injektionsanordnung umfaßt eine Flügelkanüle 100 und einen weiblichen Luer-Lock-Konneketor 12, welche mittels eines flexiblen Verbindungsschlauches 11 unter Ausbildung eines durchgängigen Lumens von dem Konnektor 12 über den Verbindungsschlauch 11 bis zur Spitze 20a der Flügelkanüle 100 verbunden sind. Die Injektionsanordnung 1 gemäß Figur 1 ist ein Einmalartikel. Die Flügelkanüle 100 setzt sich aus dem Nadelhalter 10 mit angeformten Flügeln 101a, 101b, welche in einer Ebene liegen und der in den Nadelhalter 10 eingesetzten Hohlnadel 20 zusammen. Die Hohlnadel 20 ist an ihrem aus dem Nadelhalter 10 herausragenden aktiven Teil vor Gebrauch mittels einer schlauchförmigen Schutzhülle 30 abgedeckt.

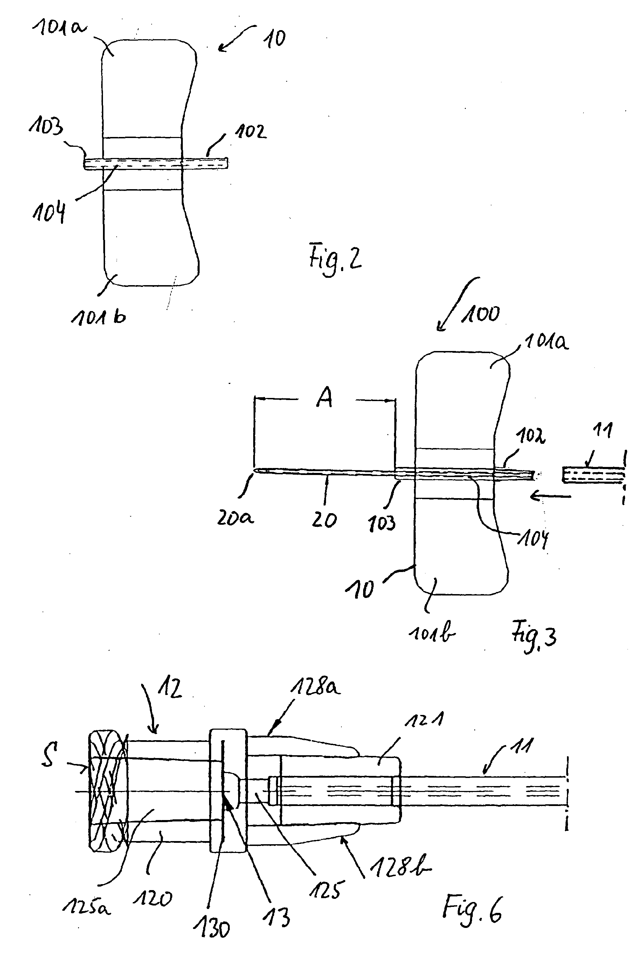

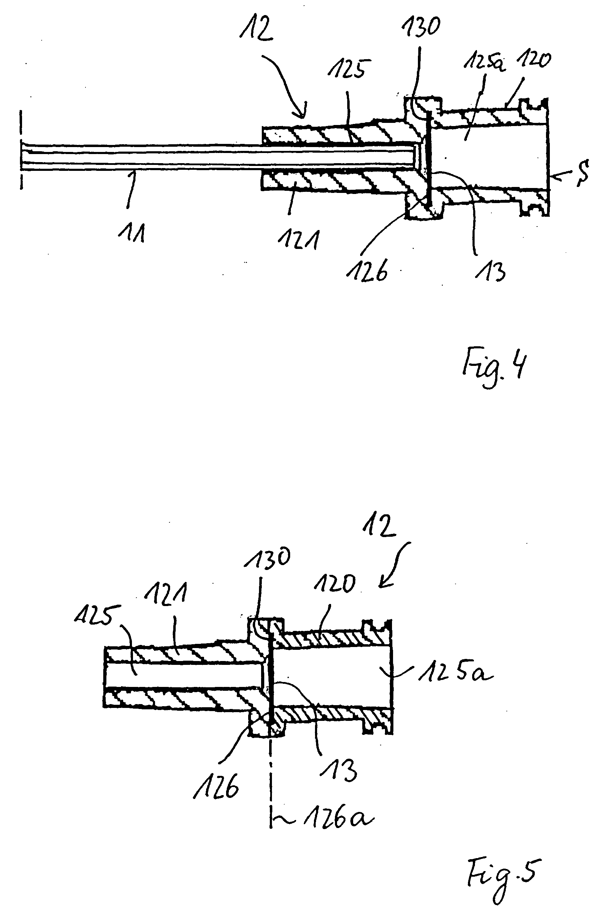

Wie aus der Figur 2 ersichtlich, weist der Nadelhalter 10 eine axial durchgehende Bohrung 104 auf, zu deren beiden Seiten sich die Flügel 101a und 101b erstrecken. Die Bohrung 104 ragt an beiden Seiten über die Flügel und den Nadelhalter mit je einem Anschlußstutzen 102 bzw. 103 vor. In den Anschlußstutzen 103 wird, wie aus der Figur 3 ersichtlich, die Hohlnadel 20 eingeführt und durch die Bohrung 104 bevorzugt bis zum Ausgang des Anschlußstutzens 102 eingeschoben, so daß die Nadel fest mit dem Nadelhalter 10 verbunden ist. Die Nadel kann auch mit dem Nadelhalter beim Spritzgießen desselben eingelegt und umspritzt werden. Die aktive Länge A der Hohlnadel beträgt etwa 15 - 30 mm, vorzugsweise etwa 18 - 20 mm, bei einem Außendurchmesser von 0,5 mm und einem Innendurchmesser von 0,28 mm. Nadelhalter 10 mit fest eingesetzter Hohlnadel 20 bilden die sogenannte Flügelkanüle 100. Auf den der Nadel gegenüber liegenden Seite des Nadelhalters wird auf den Anschlußstutzen 102 der Verbindungsschlauch 11 mit durchgehendem Lumen aufgeschoben und haftfest verbunden, beispielsweise durch Hochfrequenzschweißung. Der Nadelhalter 10 ist auch aus einem thermoplastischen Kunststoff in formstabiler Ausführung hergestellt, beispielsweise aus Weich-PVC.

Der Verbindungsschlauch hat eine bevorzugte Länge B im Bereich von 30 - 400 mm, beispielsweise 350 mm. Auf dem Verbindungsschlauch 11 kann wie in der Figur 1 ersichtlich eine Auf-Zu-Klemme 15 zum Abklemmen des Verbindungsschlauches aufgebracht sein.

An dem der Flügelkanüle 100 abgewandten Ende des Verbindungsschlauches 11 ist, wie aus der Figur 1 ersichtlich, ein weiblicher Luer-Lock-Konnektor 12 mit einer durchgehenden Bohrung vorgesehen, mit dem das Ende des Verbindungsschlauches 11 fest verbunden ist. Der Luer-Lock-Konnektor 12 ist mit einem Filterelement 13 ausgerüstet und kann bei Nichtgebrauch mittels einer Schutzkappe 14 abgedeckt sein an seinem freien Ende. Bei Inbetriebnahme wird die Schutzkappe 14 entfernt und stattdessen die mit einer Injektionslösung 50 angefüllte Spritze 40 mit ihrer z.B. als Luer-Lock ausgebildeten Spitze 43 mit dem Konnektor 12 in Verbindung gebracht.

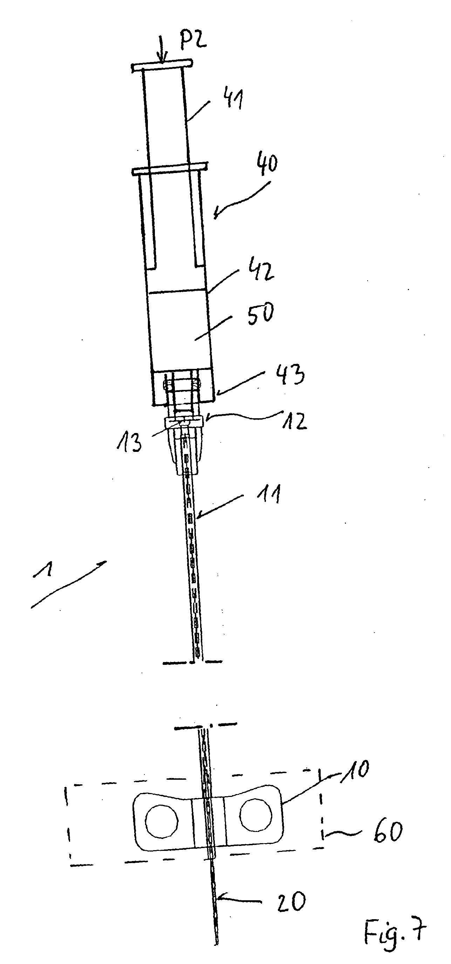

In der Figur 4 ist der Konnektor 12 mit eingesetztem Verbindungsschlauch 11 gemäß Figur 1 vergrößert dargestellt. Der weibliche Luer-Lock-Konnektor 12 weist einen Einsteckbereich 121 für die Aufnahme des Verbindungsschlauches 11 und einen sich hieran anschließenden Konnektionsbereich 120 für den Ansatz der Spritze auf. Der Konnektor 12 weist eine vom Einsteckbereich 121 bis zum Konnektionsbereich 120 axial durchgehende Bohrung 125 auf, welche sich im Übergang vom Einsteckbereich zum Konnektionsbereich erweitert - erweiterte Bohrung 125a und hierbei einen stufenförmigen Absatz 126 ausbildet. Das Filterelement ist innerhalb des Konnektors 12 in der Bohrung 125 untergebracht und zwar vorzugsweise in dem Übergangsbereich zwischen dem Einsteckbereich 121 und dem Konnektionsbereich 120, wobei das Filterelement 13 an dem stufenförmigen Absatz 126 anliegt. Das Filterelement 13 ist mit dem Konnektor dauerhaft verbunden und unverschieblich. Das Filterelement 13, das scheibenförmig, beispielsweise aus einem Gewebe aus Polyamid, wie Nylon, mit einer Porengröße 20 µm ausgebildet ist, weist einen größeren Durchmesser als die erweiterte Bohrung 125a des Konnektionsbereiches 120 auf. Der Bereich 130 des Außenumfanges des Filterelementes, der über die Bohrung 125a hinausragt, ist in den Konnektor, d.h. die Wandung des Konnektors 12 eingebettet. Der mittlere Bereich des Filterelementes 13 überspannt die durchgehende Bohrung 125, 125a des Konnektors 12 und ermöglicht das Durchströmen der mittels einer Spritze eingebrachten Injektionslösung in den Verbindungsschlauch 11. Beim Durchströmen durch das Filterelement 13 werden unerwünschte Partikelchen aus der Injektionslösung ausgefiltert und in dem Filterelement zurückgehalten, so daß sie nicht in den Blutkreislauf des Patienten gelangen können. Die Spritzenseite des Konnektors 12 ist mit S bezeichnet. Der Konnektor 12 wird aus einem thermoplastischen Kunststoff als Spritzgußteil hergestellt, wobei bevorzugt formstabile steife Kunststoffe verwendet sind. Um das Einbetten und Fixieren des Filterelementes 13 in den Konnektor zu ermöglichen, wird beispielsweise, wie aus der Figur 5 ersichtlich, der Konnektor 12 beim Herstellen aus mehreren Teilen aufgebaut, die miteinander verbunden sind. Beispielsweise wird zuerst der Einsteckbereich 121 als Spritzgußteil aus einem thermoplastischen Kunststoff wie Polycarbonat gefertigt und anschließend an der Eingangsseite 126a des Einsteckbereiches, nämlich der der Spritze zugewandten Seite das scheibenförmige Filterelement aufgelegt. Danach wird der Einsteckbereich 120 als zweites Spritzgußteil angegossen bevorzugt aus dem gleichen Werkstoff wie der Einsteckbereich 121 und hierbei wird das Filterelement entlang seines Umfanges 130 zwischen den beiden Spritzgußteilen 121 und 120 eingebettet.

In der Figur 6 ist eine weitere Ausbildung des weiblichen Luer-Lock-Konnektors 12 für die erfindungsgemäße Injektionsanordnung gemäß Figur 1 dargestellt. Der Konnektor 12 gemäß Figur 6 zeichnet sich durch zusätzliche Flügel 128a, 128b aus, die in einer Ebene an dem Einsteckbereich 121 angeformt sind.

In der Figur 7 ist der Einsatz der erfindungsgemäßen Injektionsanordnung 1 schematisch dargestellt. Die erfindungsgemäße Injektionsanordnung 1 mit Hohlnadel 20, Nadelhalter 10, Verbindungsschlauch 11 und Konnektor 12 bildet ein Teil, wobei der Verbindungsschlauch 11 sowohl fest mit dem Konnektor 12 und fest mit dem Nadelhalter 10, insbesondere unlösbar verbunden ist. Die Spritze 40 mit Spritzenzylinder 42 und Kolben 41 kann mit einem pulverförmigen Medikament vorbefüllt sein und wird unmittelbar vor der Anwendung mit sterilem Wasser aufgefüllt, so daß die mit Injektionslösung 50 angefüllte fertige Spritze 40 vorliegt. Die Spritze wird mit ihrer Spitze 43 nun direkt in den weiblichen Luer-Lock-Konnektor 12 eingeführt in die erweiterte Bohrung 125a und die Injektionslösung kann durch Drücken des Kolbens 41 in Pfeilrichtung P2 durch den Konnektor, den Verbindungsschlauch 11 und die Hohlnadel 20 dem Patienten injiziert werden. Die Injektionsanordnung 1 kann mittels eines Pflasters 60 an der Hautoberfläche des Patienten nahe der Injektionsstelle fixiert werden. Das Filterelement 13 in dem Konnektor 12 hält zuverlässig Feststoffteilchen, welche mit der Injektionslösung in den Konnektor gelangen zurück, so daß auch bei Auffüllung der Spritze 40 mit Injektionslösung oder sterilem Wasser keine unerwünschten Feststoffteilchen mit der Injektionslösung in den Blutkreislauf gelangen können.

Die Erfindung ermöglicht das einfache Handling bei Injektion mittels einer Spritze, insbesondere von pulverförmigen Medikamenten, die erst noch mit sterilem Wasser gelöst werden müssen, auch durch den Patienten und in Notfallsituationen. Durch die erfindungsgemäße Injektionsanordnung mit Flügelkanüle, Verbindungsschlauch sowie Konnektor mit eingebautem Filterelement werden Feststoffpartikel aus der Injektionslösung mit Sicherheit entfernt werden.

## Patentansprüche

1. Injektionsanordnung zum Injizieren von Injektionslösungen, welche aus pulverförmigen Arzneimitteln unmittelbar vor Gebrauch in sterilem Wasser zubereitet und mittels einer Spritze verabreicht werden, umfassend eine Flügelkanüle (100) mit einer Hohlnadel (20) und einem Nadelhalter (10) mit Flügeln sowie einen mit der Flügelkanüle (100) mittels eines flexiblen Verbindungsschlauches (11) verbundenen weiblichen Luer-Lock-Konnektor (12) mit einem Einsteckbereich (121) für den Verbindungsschlauch (11) und einem Konnektionsbereich (120) für das Ansetzen der mit Injektionslösung (50) gefüllten Spritze (40) und einer vom Einsteckbereich (121) bis zum Konnektionsbereich (120) des Luer-Lock-Konnektors (12) durchgehenden Bohrung (125), in die ein für die Injektionslösung (50) durchlässiges poröses Filterelement (13) eingesetzt ist, **dadurch gekennzeichnet, daß** der weibliche Luer-Lock-Konnektor (12) mit dem Filterelement (13) integral verbunden ist, wobei ein erstes, den Einsteckbereich (121) bildendes Spritzgußteil aus einem thermoplastischen Kunststoff vorgesehen ist, auf dessen Eingangsseite (126a) das Filterelement (13) aufgelegt und durch Aufspritzen eines zweiten, den Konnektionsbereich (120) bildenden Spritzgußteils aus dem gleichen oder einem anderen thermoplastischen Kunststoff wie der Einsteckbereich (121) eingebettet ist und beide Spritzgußteile zu dem Luer-Lock-Konnektor (12) vereinigt sind.

2. Injektionsanordnung nach Anspruch 1, **dadurch gekennzeichnet, daß** das Filterelement (13) Poren mit einem mittleren Durchmesser von 2 - 100 µm aufweist.

3. Injektionsanordnung nach einem der Ansprüche 1 oder 2, **dadurch gekennzeichnet, daß** das Filterelement (13) Poren mit einem mittleren Durchmesser von 5 - 20 µm aufweist.

4. Injektionsanordnung nach einem der Ansprüche 1 bis 3, **dadurch gekennzeichnet, daß** das Filterelement (13) im Übergang vom Einsteckbereich (121) zum Konnektionsbereich (120) des Luer-Lock-Konnektors (12) angeordnet ist, wobei die durchgehende Bohrung (125) sich im Übergang zum Konnektionsbereich (120) erweitert und einen stufenförmigen Absatz (126) bildet.

5. Injektionsanordnung nach einem der Ansprüche 1 bis 4, **dadurch gekennzeichnet, daß** das Filterelement (13) entlang seines Außenumfanges in dem Luer-Lock-Konnektor (12) eingelagert ist.

6. Injektionsanordnung nach einem der Ansprüche 1 bis 5, **dadurch gekennzeichnet, daß** als Filterelement (13) ein Gewebe auf Basis von Polyamid vorgesehen ist.

7. Injektionsanordnung nach einem der Ansprüche 1 bis 5, **dadurch gekennzeichnet, daß** als Filterelement eine Scheibe aus gesintertem Kunststoffgranulat oder Metallpulver vorgesehen ist.

8. Injektionsanordnung nach einem der Ansprüche 1 bis 7, **dadurch gekennzeichnet, daß** der Verbindungsschlauch (11) aus einem flexiblen Kunststoff hergestellt ist und eine Länge von 30 - 400 mm aufweist.

9. Injektionsanordnung nach einem der Ansprüche 1 bis 8, **dadurch gekennzeichnet, daß** der Verbindungsschlauch (11) einen Außendurchmesser von 1,0 - 2,5 mm und einen Innendurchmesser von 0,2 - 1,5 mm aufweist.

10. Injektionsanordnung nach einem der Ansprüche 1 bis 9, **dadurch gekennzeichnet, daß** der Luer-Lock-Konnektor (12) an seinem Einsteckbereich (121) außenseitig zwei in einer Ebene liegende angeformte Flügel (128a, 128b) aufweist.

11. Injektionsanordnung nach einem der Ansprüche 1 bis 10, **dadurch gekennzeichnet, daß** der Luer-Lock-Konnektor (12) aus einem thermoplastischen Kunststoff wie Acrylbutadienstyrol, Polycarbonat oder Polymethacrylat gefertigt ist.

12. Injektionsanordnung nach einem der Ansprüche 1 bis 11, **dadurch gekennzeichnet, daß** der aktive Bereich der Hohlnadel (20) der Flügelkanüle (100) eine Länge (A) von 15 - 30 mm aufweist.

13. Injektionsanordnung nach einem der Ansprüche 1 bis 12, **dadurch gekennzeichnet, daß** die Hohlnadel (20) mit einer abziehbaren Schutzhülle (30) versehen ist.

14. Injektionsanordnung nach einem der Ansprüche 1 bis 13, **dadurch gekennzeichnet, daß** der Nadelhalter (10) der Flügelkanüle (100) mit einem Abdeckungsschutz zum Abdecken der Spitze der Hohlnadel (20) nach Gebrauch ausgestattet ist.

## Claims

1. An injection system for injecting injection solutions that are prepared from powdered medicaments in sterile water immediately before use and are administered by means of a syringe, comprising a winged cannula (100) with a hollow needle (20) and a needle holder (10) with wings, and a female Luer lock connector (12) which is connected to the winged cannula (100) by means of a flexible connecting tube (11) and has an insertion region (121) for the connecting tube (11) and a connection region (120) for attachment of the syringe (40) filled with injection solution (50), and having a continuous bore (125) which extends from the insertion region (121) to the connection region (120) of the Luer lock connector (12) and into which a porous filter element (13) permeable to the injection solution (50) is fitted, **characterized in that** the female Luer lock connector (12) is integrally connected to the filter element (13), a first thermoplastic injection-moulded part forming the insertion region (121), the filter element (13) being placed on the inlet side (126a) thereof and being embedded by spraying-on of a second injection-moulded part that forms the connection region (120) and is made of the same thermoplastic as the insertion region (121) or of a different thermoplastic, and both injection-moulded parts being joined together to form the Luer lock connector (12).

2. An injection system according to claim 1,
**characterized in that** the filter element (13) has pores with a mean diameter of 2 - 100 µm.

3. An injection system according to either of claims 1 and 2, **characterized in that** the filter element (13) has pores with a mean diameter of 5 - 20 µm.

4. An injection system according to any one of claims 1 to 3, **characterized in that** the filter element (13) is arranged at the transition from the insertion region (121) to the connection region (120) of the Luer lock connector (12), the continuous bore (125) widening at the transition to the connection region (120) and forming a step-shaped shoulder (126).

5. An injection system according to any one of claims 1 to 4, **characterized in that** the filter element (13) is embedded along its outer circumference in the Luer lock connector (12).

6. An injection system according to any one of claims 1 to 5, **characterized in that** a woven fabric based on polyamide is provided as filter element (13).

7. An injection system according to any one of claims 1 to 5, **characterized in that** a disc of sintered plastic granulate or metal powder is provided as filter element.

8. An injection system according to any one of claims 1 to 7, **characterized in that** the connecting tube (11) is made of a flexible plastic and has a length of 30 - 400 mm.

9. An injection system according to any one of claims 1 to 8, **characterized in that** the connecting tube (11) has an external diameter of 1.0 - 2.5 mm and an internal diameter of 0.2 - 1.5 mm.

10. An injection system according to any one of claims 1 to 9, **characterized in that** the Luer lock connector (12) has, on the outside of its insertion region (121), two integrally formed wings (128a, 128b) lying in one plane.

11. An injection system according to any one of claims 1 to 10, **characterized in that** the Luer lock connector (12) is made of a thermoplastic such as acrylonitrile-butadiene-styrene, polycarbonate or polymethacrylate.

12. An injection system according to any one of claims 1 to 11, **characterized in that** the active region of the hollow needle (20) of the winged cannula (100) has a length (A) of 15 - 30 mm.

13. An injection system according to any one of claims 1 to 12, **characterized in that** the hollow needle (20) is provided with a removable protective sheath (30).

14. An injection system according to any one of claims 1 to 13, **characterized in that** the needle holder (10) of the winged cannula (100) is equipped with a protective cover for covering the tip of the hollow needle (20) after use.

## Revendications

1. Système d'injection pour injecter des solutions d'injection qui sont préparées dans de l'eau stérile à partir de médicaments en poudre immédiatement avant usage et sont administrées au moyen d'une seringue, comprenant une canule à ailettes (100) avec une aiguille creuse (20) et un porte-aiguille (10) à ailettes ainsi qu'un connecteur Luer-Lock femelle (12) relié à la canule à ailettes (100) au moyen d'un tuyau souple d'accouplement (11) flexible et comprenant une région d'emboîtement (121) pour le tuyau souple d'accouplement (11) et une région de connexion (120) pour la mise en place de la seringue (40) remplie de solution d'injection (50) et un orifice de passage (125) allant de la région d'emboîtement (121) jusqu'à la région de connexion (120) du connecteur Luer-Lock (12) dans lequel est inséré un élément filtrant (13) poreux laissant passer la solution d'injection (50), **caractérisé en ce que** le connecteur Luer-Lock femelle (12) est intégralement relié à l'élément filtrant (13) du fait qu'il est prévu une première pièce moulée par injection en matière synthétique thermoplastique, qui forme la région d'emboîtement (121), à laquelle est intégrée l'élément filtrant (13) placé sur le côté entrée (126a) de celle-ci en appliquant une deuxième pièce moulée par injection dans la même ou une autre matière synthétique thermoplastique que la région d'emboîtement (121), qui forme la région de connexion (120), et que les deux pièces moulées par injection sont assemblées pour former le connecteur Luer-Lock (12).

2. Système d'injection selon la revendication 1, **caractérisé en ce que** l'élément filtrant (13) présente des pores avec un diamètre moyen de 2 - 100 µm.

3. Système d'injection selon la revendication 1 ou 2, **caractérisé en ce que** l'élément filtrant (13) présente des pores avec un diamètre moyen de 5 - 20 µm.

4. Système d'injection selon l'une des revendications 1 à 3, **caractérisé en ce que** l'élément filtrant (13) est disposé au niveau de la transition de la région d'emboîtement (121) à la région de connexion (120) du connecteur Luer-Lock (12), l'orifice de passage (125) s'élargissant au niveau de la transition à la région de connexion (120) et formant un épaulement (126) en forme de gradin.

5. Système d'injection selon l'une des revendications 1 à 4, **caractérisé en ce que** l'élément filtrant (13) est placé le long de sa périphérie extérieure dans le connecteur Luer-Lock (12).

6. Système d'injection selon l'une des revendications 1 à 5, **caractérisé en ce qu'**il est prévu un tissu à base de polyamide comme élément filtrant (13).

7. Système d'injection selon l'une des revendications 1 à 5, **caractérisé en ce qu'**il est prévu un disque en granulés de plastique ou en poudre métallique fritté comme élément filtrant.

8. Système d'injection selon l'une des revendications 1 à 7, **caractérisé en ce que** le tuyau souple d'accouplement (11) est fabriqué en matière synthétique flexible et présente une longueur de 30 - 400 mm.

9. Système d'injection selon l'une des revendications 1 à 8, **caractérisé en ce que** le tuyau souple d'accouplement (11) présente un diamètre extérieur de 1,0 - 2,5 mm et un diamètre intérieur de 0,2-1,5 mm.

10. Système d'injection selon l'une des revendications 1 à 9, **caractérisé en ce que** le connecteur Luer-Lock (12) présente sur l'extérieur de la région d'emboîtement (121) deux ailes (128a, 128b) formées sur celle-ci et se trouvant dans un plan.

11. Système d'injection selon l'une des revendications 1 à 10, **caractérisé en ce que** le connecteur Luer-Lock (12) est réalisé dans une matière synthétique thermoplastique telle que du acrylonitrile-butadiène-styrène, du polycarbonate ou du polyméthacrylate.

12. Système d'injection selon l'une des revendications 1 à 11, **caractérisé en ce que** la région active de l'aiguille creuse (20) de la canule à ailettes (100) présente une longueur (A) de 15 - 30 mm.

13. Système d'injection selon l'une des revendications 1 à 12, **caractérisé en ce que** l'aiguille creuse (20) est pourvue d'une gaine de protection amovible (30).

14. Système d'injection selon l'une des revendications 1 à 13, **caractérisé en ce que** le porte-aiguille (10) de la canule à ailettes (100) est équipé d'une protection de recouvrement pour recouvrir la pointe de l'aiguille creuse (20) après usage.
